# EUROPEAN PATENT SPECIFICATION

(11) **EP 4 252 002 B1**
(45) Date of publication and mention of the grant of the patent: **20.03.2024**
(21) Application number: 22744154.0
(22) Date of filing: 01.07.2022
(51) Int. Cl.: G01N 33/543, G01N 33/68, G01N 33/74, G01N 33/76

(54) **A REUSABLE TEST DEVICE**
EIN WIEDERVERWENDBARES TESTGERÄT
UN APPAREIL DE TEST RÉUTILISABLE

(30) Priority: 02.07.2021 SE 2150868
(43) Date of publication of application: 04.10.2023
(73) Proprietor: PHARMISTA TECHNOLOGIES AB, 223 81 Lund (SE)
(72) Inventor: MATTSSON, Alice Anna Lovisa, 116 33 Stockholm (SE); PORTER, Robert Andrew, Aberdeenshire NN10 9LF (GB)
(74) Representative: Høiberg P/S
(86) International application number: PCT/EP2022/068337
(87) International publication number: WO 2023/275389

(56) References cited:
- WO-A1-2013/033541
- WO-A2-2005/098439
- US-A1- 2017 227 486
- CAMPUZANO SUSANA ET AL: "New challenges in point of care electrochemical detection of clinical biomarkers", SENSORS AND ACTUATORS B: CHEMICAL, ELSEVIER BV, NL, vol. 345, 25 June 2021 (2021-06-25), XP086731876, ISSN: 0925-4005, DOI: 10.1016/J.SNB.2021.130349 [retrieved on 2021-06-25]
- TORRINI FRANCESCA ET AL: "Sensitive 'two-steps' competitive assay for gonadotropin-releasing hormone detection via SPR biosensing and polynorepinephrine-based molecularly imprinted polymer", ANALYTICA CHIMICA ACTA, ELSEVIER, AMSTERDAM, NL, vol. 1161, 6 April 2021 (2021-04-06), XP086550755, ISSN: 0003-2670, DOI: 10.1016/J.ACA.2021.338481 [retrieved on 2021-04-06]

## Description

### FIELD OF THE INVENTION

The present invention relates to a reusable test device comprising molecular imprinted polymers (MIPs), a method for testing the presence of an analyte in a fluid, and use of the reusable test device for pregnancy testing.

### BACKGROUND

An immunoassay (IA) test is a biochemical test which is able to detect and locate the presence of a macromolecule or a small molecule, referred to as an "analyte". The analyte can be a number of items that come from the human body. Normally, an immunoassay measures the presence or concentration of an analyte in a solution through the use of an antibody.

An example of an immunoassay test is a pregnancy test. In a pregnancy test, the pregnancy marker human chorionic gonadotropin (hCG) is detected. hCG is a hormone that is expressed by placental trophoblasts during pregnancy and comprises α and β-subunits (hCGα & hCGβ) which both may be used as pregnancy markers. Studies have shown that the molar ratios of beta hCG to alpha hCG and beta hCG to hCG may be higher in early gestation, and a reversal of the beta hCG to alpha hCG ratio at 12-13 weeks gestation.

Common pregnancy tests on the market today are in the form of Lateral Flow Assay (LFA), also known as Lateral Flow Immunochromatographic Assay (LFIA). Such tests typically comprise a paper-based platform for the detection of hCG target analytes in liquid samples, as well as a plastic casing surrounding the paper. The sample is placed on the paper and allowed to flow along a solid porous matrix by capillary action. Typically, these pregnancy tests comprise an antibody that binds the hCG target analyte.

However, current pregnancy tests are limited to disposable use as a consequence of that removal of the analyte form the antibody results in degradation of the antibody. Hereby, the affinity and selectivity for the hCG target analyte is degenerated.

Moreover, current pregnancy tests allow for interpretation. Research shows that 25 % of women have difficulties with interpreting the lines of a traditional pregnancy test. To exemplify this, a thread called "for those of us who needs help analyzing pregnancy tests" has gained 224 000 posts since 2011 in Sweden alone. This figure may help to explain why 45 % of women prefer to purchase tests that display the results digitally (which are to be mentioned based on the exact same LFA method), although they might refrain from doing so since they are much more expensive.

Another problem with the current tests being sold is that they are not as accurate as they claim to be. This is due to the "99 % accurate in detecting pregnancies" label only explains that the product is 99 % accurate in comparison to other hCG tests, based on information submitted to the FDA back in 1976. This is a part of the explanation as to why 50 % of the pregnancy tests sold in Germany in 2014 where not as accurate in detecting pregnancies as the package claimed, since they were not able to detect low levels of hCG.

In regard to psychological aspects, customers explain that it can be both stressful and mentally draining not having a pregnancy test within reach if signs of pregnancy would occur. Women partaking in qualitative studies about infertility has also mentioned that for every time they had to return to the store to purchase another bulk of pregnancy tests it is a proof of them not being able to conceive, which creates a lot of negative emotions. This can in long term contribute to worsening the odds of becoming pregnant, since it is known that stress may affect fertility negatively.

Lastly, since most of the tests currently being sold has a plastic casing around them, they contribute to environmental degradation. For instance, it is estimated that 900 tons of waste generated by pregnancy tests ends up in landfill each year as a result of 8 out of 10 not recycling their pregnancy
tests correctly.

WO 2013/033541 discloses a sensor for the detection of a waterborne target molecule comprising a molecularly imprinted polymer film, and MIP powders for removal of waterborne target molecules through the use of, for example, a flow cell. The methods involve using the target molecule in the preparation of the MIP films and powders. When the target molecule is removed, it leaves behind an MIP with cavities that are complementary in shape and functionality to the target molecule. The MIP thereby created can bind target molecules in those cavities.

There is a need for an improved test device which is able to be reused, easier to interpret, more accurate, and more environmentally friendly than to those described in the prior art.

### SUMMARY OF INVENTION

An object of the inventive concept is to mitigate the above problems, and to provide a reusable test device that can be re-used upon removal of the analyte from the binding moiety. A further object is to provide a reusable test device with improved shelf life, and thus more environmentally friendly, than prior art solutions. Still a further object is to provide an improved method which enables repeated tests for the presence of an analyte in a fluid.

According to a first aspect of the invention, the above objects are achieved by a reusable test device comprising:
a) a wick adapted to collect a fluid to be analyzed;
b) a reusable sensor unit, comprising:
   a MIP layer comprising at least one type of molecular imprinted polymers (MIPs) adapted to bind at least one analyte present in the fluid;
   an electrode layer comprising at least one electrode,
   wherein the reusable sensor unit is regenerable upon cleansing, and
c) a rechargeable electronic unit adapted to read out results from the reusable sensor unit.

The wick is detachably connected to the reusable sensor unit, and the reusable sensor unit may be detachably connected to the rechargeable electronic unit.

The present inventors surprisingly found that Molecularly Imprinted Polymers (MIPs) may be used as a replacement for conventional binding moieties, such as antibodies, in a re-usable immunoassay format which comprises a reusable sensor unit, since antibodies cannot be re-used as the process to remove the analyte requires conditions that may damage or destroy the binding moiety of the antibody. The reusable test device may therefore have a reusable sensor unit that contains no antibodies. In particular the test device may have a reusable sensor unit which contains no other binding moieties than MIP. The use of MIPs is favorable because it may increase the structural integrity, so when the reusable sensor unit is regenerated after a binding event the binding affinity and selectivity may remain mostly unchanged, allowing for that reusable sensor to be reused. The test device may additionally have the capacity to retain a shelf life in the order of many years, as well as the capability to be regenerated with a wash treatment.

MIPs are made by allowing functional and cross-linking monomers of plastic materials, such as methacrylic and styrene, to interact with a templating ligand to create low-energy interactions. During subsequent polymerization, the molecule of interest is entrapped within the polymer either by a non-covalent, self-assembling approach, or by a reversible, covalent approach. After stopping the polymerization, the template molecule is washed out. The resultant imprint of the template is maintained in the rigid polymer and possesses a steric (size, shape) and chemical (special arrangement of complementary functionality) memory for the template. The MIPs may be able to bind the template, i.e. the analyte, with a specificity similar to that of an antigen-antibody interaction. Identical or similar to the analyte (target molecule) to detect.

The MIP may have a better binding strength if the MIP is a ridged structure. However, a rigid structure could make the regeneration of the MIP difficult. The choice of monomer units will create the intermolecular interactions between the MIP and the target used, thus creating some specificity to sticking to the target. The crosslinker will allow for the structure shape to allow that target molecule, i.e. analyte, to enter the polymer. This often described as a lock and key scenario where the MIP has to have the right charge and shape to accept the key of the target molecule to allow just that molecule to successfully bind and no other.

The dynamic range of the test device may depend on the number of active sites (MIPs) on layer (7) and the binding strength between the MIP and the analyte. Intermolecular interactions between the MIP and the analyte are stronger when there are lots of these interactions going on.

As used herein, the term "analyte" is referred to the target molecule which the MIP is adapted to bind. The at least one analyte may comprise a hormone, the hormone being selected from the group consisting of estrogen, progesterone, luteinizing hormone (LH), and human chorionic gonadotropin (hCG), such as hyperglycosylated human chorionic gonadotropin (hCG-H), in particular beta human chorionic gonadotropin (β-hCG). In particular, the at least one type of molecular imprinted polymer may be adapted to bind hCG.

As used herein, the term "fluid" refers to any liquid. The fluid may comprise a body fluid, such as saliva, blood, and urine, preferably urine.

The test device may have the form of a bench top or small handheld unit, making it portable and easy to use and transport.

According to a second aspect of the inventive concept the reusable test device is used for pregnancy testing. In pregnancy tests, normally hormone beta human chorionic gonadotropin (β-hCG) is detected. Alternatively, the reusable test device may be used for fertility testing, in vitro fertilization, multiple children testing, or predicting miscarriage, as hCG levels that do not follow the normal trend of doubling or tripling every day could lead to an issue with the pregnancy. The LH-level is important for determining a good time for implantation in in vitro fertilization (IVF), low levels of LH could lead to low success rates for implantation. High levels of LH could be an indication of polycystic ovary syndrome, which is a common cause of infertility issue.

The MIP layer of the reusable sensor unit may comprise two or more types of MIPs, such as three or four types of MIPs. Hereby, the MIP layer may comprise a first type of MIPs adapted to bind a first analyte, and a second type of MIPs, adapted to bind the first analyte and/or a second analyte. This may be beneficial since the test device may have a higher accuracy. The reusable test device may further comprise a third type of MIPs adapted to bind any of the first, second and/or a third analyte.

Whether or not the reusable test device is intended to be used for pregnancy testing, the first analyte may be hCG and the second analyte glycosylated hCG or progesterone. Hereby, the first type of molecular imprinted polymer may be adapted to bind hCG, and the second type of molecular imprinted polymer may be adapted to bind glycosylated hCG or progesterone.

As mentioned above, the test device is beneficial with regards to sustainability since the individual components may be re-used. The wick may, however, be a disposable wick. Hence, the wick may be the only disposable item of the test device. The wick may for example be a paper strip.

The purpose of the wick is primarily to collect the fluid. However, the wick may also have a delivering function. For example, the wick may be adapted to deliver fixed concentrations of salts and/or electroactive material to layer (7). For that purpose, the wick may comprise a buffer solution, such as phosphate, Tris, or citrate based buffer. The concentration of such buffer solution may be from 0.1 mM to 100 mM, such as 0.5 to 75 nM.

It should be noted that the MIP layer and the electrode layer may together form a single layer. Hereby, the MIP layer and the electrode layer may together form an integral layer, e.g. through polymerization, absorption, or co-incorporation into a polymer surface or paste.

The measurement process for the reusable test device may be electrical,
electrochemical, optical, and/or plasmonic, as will be described below.

An electrochemically based measurement method may be accomplished by the monomer units making up the MIP being electroactive. A binding event of the analyte will then disrupt the electrochemistry or electroactivity of the MIP polymer structure in a measurable way. Alternatively, a physical property associated with the presence of an analyte in the MIP may be a change in the resistance of the MIP layer with or without the analyte bound.

An optical based measurement method may be accomplished by a colored monomer unit being added into the MIP polymer structure. When the target analyte binds into the MIP, this causes a charged and conformational change in the polymer, which would result in a change in the colour of the polymer.

A surface plasmon resonance (SPR) measurement method may be accomplished by a resonance frequency being passed through the MIP layer, causing vibrations due to electrical oscillation. This vibration may be measured and if the mass of the MIP layer changes (due to the analyte binding in the MIP) the oscillation frequency or amplitude is disrupted as a result in a measurable way.

According to a further aspect of the inventive concept, a method for testing the presence of an analyte in a fluid is provided. The method comprises the steps:
providing (S1) a reusable test device of the invention;
collecting (S2) a fluid on the wick;
analyzing (S3) the fluid on the wick;
removing (S4) the wick from the reusable sensor unit, and
washing (S5) the reusable sensor unit with at least one washing medium to remove any bound analytes from the molecular imprinted polymers, wherein the steps S1 to S5 may be repeated. The test device is then ready to be used once again with a new sample collected on a wick, preferably a new wick. The method may be used for pregnancy testing.

The step of collecting is preferably performed with the wick connected to the reusable sensor unit, and with the reusable sensor unit connected to the rechargeable electronic unit, in an assembled state.

The step of washing may be performed by a washing medium comprising a liquid.

The washing medium may comprise a liquid, such as tap water, e.g. hot tap water, or an aqueous based liquid comprising a surfactant and or a salt.

The washing step may be performed by the user herself or by nursing staff. A container with washing medium may be provided to the user together with the test device.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of the element, device, component, means, step, etc., unless explicitly stated otherwise. The steps of any method disclosed herein do not have to be performed in the exact order disclosed, unless explicitly stated.

### BRIEF DESCRIPTION OF THE DRAWINGS

The disclosure will be described in more detail with reference to the appended schematic drawings, which show an example of a presently preferred embodiment of the disclosure.
Fig. 1a illustrates a reusable test device according to a first embodiment of the invention.
Fig. 1b an exploded view of the reusable test device of Fig. 1a.
Figs. 2a and b illustrates a perspective view of a MIP layer comprising MIPs according an embodiment.
Fig. 3 illustrates a flow chart of a method of use of the reusable test device in Figs. 1a and 1b.
Fig. 4 illustrates displacement of FITC-doped SV-imprinted MIP nanoparticles (SV-MIP) bound to SV-modified magnetic nanoparticles (SV-magNP) upon addition of different peptides (SV (SEQ ID NO:1): SIRI,PGCPRGVNPVV; PQ (SEQ ID NO:2): PGPSDTPILPQ; QK (SEQ ID NO:3): QKSLSLSPGK)
Fig. 5 illustrates quartz crystal microbalance (QCM) frequency change during addition of SV-imprinted MIP nanoparticles (SV-MIP) to an SV-modified sensor chip. Apparent dissociation constant, KD < 1 nM.
Fig. 6 illustrates change in SPR response units (RUs) upon addition of an SV-NG and a nonimprinted NG (NIP-NG) to an SV modified sensor chip. The apparent dissociation constant was KD = 11 nM.

### DETAILED DESCRIPTION OF THE INVENTION

The present disclosure will now be described more fully hereinafter with reference to the accompanying drawings, in which currently preferred embodiments of the disclosure are shown. The present disclosure may, however, be embodied in many different forms and should not be construed as limited to the embodiments set forth herein; rather, these embodiments are provided for thoroughness and completeness, and to fully convey the scope of the disclosure to the skilled addressee. Like reference characters refer to like elements throughout.

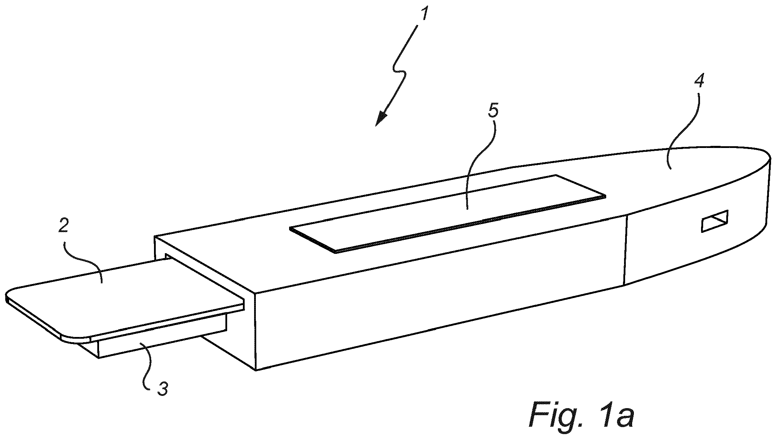

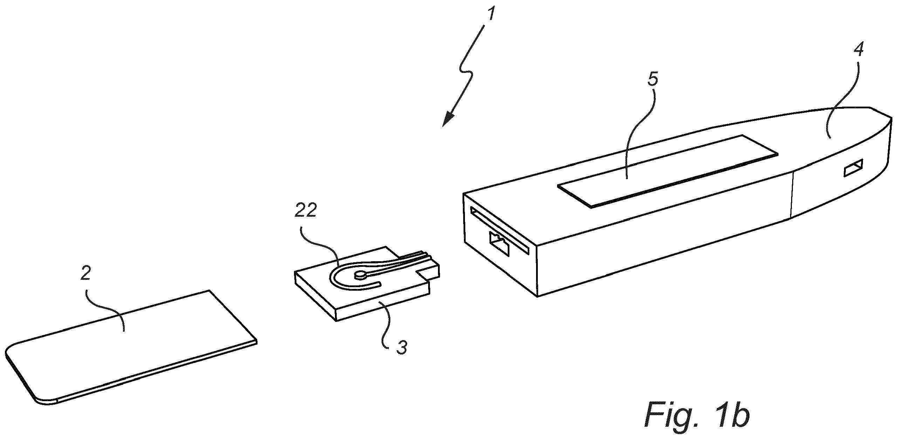

Figs. 1a and 1b show a reusable test device 1 that comprises a wick 2, a reusable sensor unit 3, and a rechargeable electronic unit 4. The reusable test device further comprises an electronic display 5. In Fig. 1a, the reusable test device 1 is illustrated in an assembled state and in Fig. 1b the reusable test device 1 is illustrated in a disassembled state. The reusable sensor unit 3 is regenerable upon cleansing, which means that the reusable test device 1 may be utilized a plurality of times, such as 10-20 times.

The wick 2, which preferably is disposable, is adapted to collect a fluid to be analyzed. The fluid may be a liquid and may comprise a body fluid, such as saliva, blood, and urine. Preferably, the wick 2 is provided on top of the sensor unit 3 which enables the analyte 10 to be forced into the sensor unit 3 by means of gravity. The wick 2 may be attached to the reusable sensor unit 3 by means of snap attachment.

In one embodiment, the wick 2 may consist essentially of cellulose, but may also comprise thermoplastics. The wick 2 may for example be a paper strip.

In addition to collecting the fluid, the wick 2 may also be adapted to deliver fixed concentrations of salts and/or electroactive material to electrode layer 7. The wick 2 may thus comprise a buffer solution, such as phosphate, Tris, citrate, or other buffering solutions (see Table 1). The concentration of the buffer solution may be from 0.1 to 100 mM. Hereby, the wick 2 may not only be adapted to collect a fluid, it may also be adapted to deliver a buffer solution comprising key chemicals to stabilize different levels of salt concentrations in the fluid. In particular, this may be beneficial when the fluid is urine since urine fluctuates in its salt concentration depending on which time of the day it is collected. From an electroanalytical point of view, it may therefore be advantageous to standardize the urine. Furthermore, electroanalytical measurements may suffer from the concentration of ions being too low which results in the curves, e.g. responsive measurement curves to the binding event, either changes in elevator activity or plasmonic function, being very small. The wick 2 may be provided with the buffer solution contained, or with the buffer solution separate in a container for e.g. dipping.

The reusable sensor unit 3 comprises at least two layers; the electrode layer 7 and the MIP layer 8. MIP layer 8 serves as a measurement layer and comprises at least one type of MIPs adapted to bind at least one analyte 10 present in the fluid. This means, the MIPs have a binding affinity for the analyte 10 and functions as a binding moiety for the analyte 10. The reusable sensor unit 3 may not contain any other binding moieties than MIPs. Thus, the sensor unit 3 may not contain any antibodies.

The analyte 10 of interest may be a hormone, such as a hormone selected from the group consisting of estrogen, progesterone, luteinizing hormone (LH), and human chorionic gonadotropin (hCG), such as hyperglycosylated human chorionic gonadotropin (hCG-H), in particular beta human chorionic gonadotropin (β-hCG)

The MIP layer 8 may comprise two or more types of MIPs, such as three or four types of MIPs. When the MIP layer 8 comprises two or more types of MIPs, each type of MIPs may be adapted to bind the same analyte 10 and/or a different analyte 10. The test device 1 may thus comprise a first type of MIPs adapted to bind a first analyte 10, and a second type of MIPs, adapted to bind the first analyte 10 and/or a second analyte 10. The first analyte 10 may be beta human chorionic gonadotropin (β-hCG). The first type of MIPs may hereby be adapted to bind hCG and the second type of MIPs may be adapted to bind glycosylated hCG.

MIPs adapted for diagnostic applications are preferably nanoparticles or nanogels produced by precipitation, emulsion or graft polymerization, and thin film materials that exhibit more homogenous binding sites due to the spatial restrictions imposed by the limited film thickness or microgel radius. Disperse phase imprinting is the preferred method for high yielding synthesis of epitope imprinted nanoparticles targeting hCG. Epitope imprinting relies on the identification of solvent exposed proteotypic epitopes that can serve as templates to generate protein specific MIPs. Overall, preferred epitopes are linear solvent exposed, either C- or N- terminal sequences, or internal conformationally defined loop structures. Moreover, the epitopes should be free from post-translational modifications and generate sufficient affinity and specificity for the target hormone i.e. allowing detection in the range 10 pM - 1 µM and absence of crossreactivity with lutenizing hormone (LH). Possible epitopes used to generate MIPs are given in Table 2. Preferred sequences demonstrated to impart specicity over lutenizing hormone are the 15 amino acid hCG beta chain sequence 66-80 (SIRLPGCPRGVNPVV = SV (SEQ ID NO:1)) and the C-terminal sequence 135-145 (PGPSDTPILPQ = PQ (SEQ ID NO:2)) (JOURNAL OF BIOLOGICAL CHEMISTRY VOL. 286, NO. 28, pp. 25016-25026, July 15, 2011). These sequences were used as templates to prepare MIP nanoparticles (see Example 1).

MIP layer 8 is preferably provided adjacent and on top of the electrode layer 7. However, the MIP layer 8 could instead be provided besides the electrode layer 7. The electrode layer 7 may comprise at least one electrode 22, such as two or three electrodes 22. The at least one electrode 22 in the electrode layer 7 may be embedded in the electrode layer 7 or provided on a surface of the electrode layer 7. The distance between the electrodes 22 may be from 1 µm to 5 mm.

When the fluid contains an analyte 10 to which the MIP has an affinity, the analyte 10 is bound to the MIP and a binding event has occurred. This binding event may result in a change in physical properties which is measurable. resistance which is measurable. The functionality may, for example, depend upon detecting differences in the resistivity of MIP layer 8 as a function of the adsorption of the analyte 10, i.e. the target molecule. Alternatively, the functionality may, for example, depend upon detecting differences in mass change. The measurement process for the reusable diagnostic may be electrical, electrochemical, optical or plasmonic.

The rechargeable electronic unit 4 is adapted to read out results from the reusable sensor unit 3. The rechargeable electronic unit 4 may comprise a digital display, which may be used to tell the user if all analyte 10 has been washed away or not. The rechargeable electronic unit 4 may comprise a connecting port for charging of data transfer. The connecting port may be a USB port.

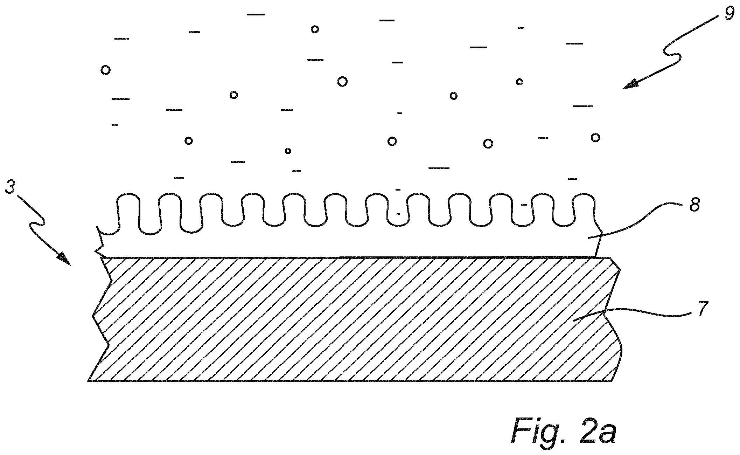

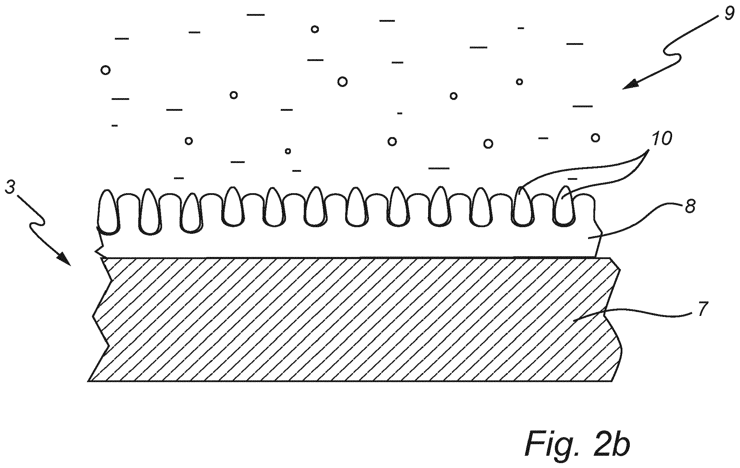

Fig. 2a illustrates a layer of MIPs and a free ion solution. Without any analyte 10 bound to the MIPs the ions solution 9 can move in and out of the MIP matrix freely. In Fig. 2b the target analyte 10 is bound into the MIP cleft whereby resistance to the MIP layer 8 is created. This will prevent the movement of free ions 9 resulting in a drop in signal.

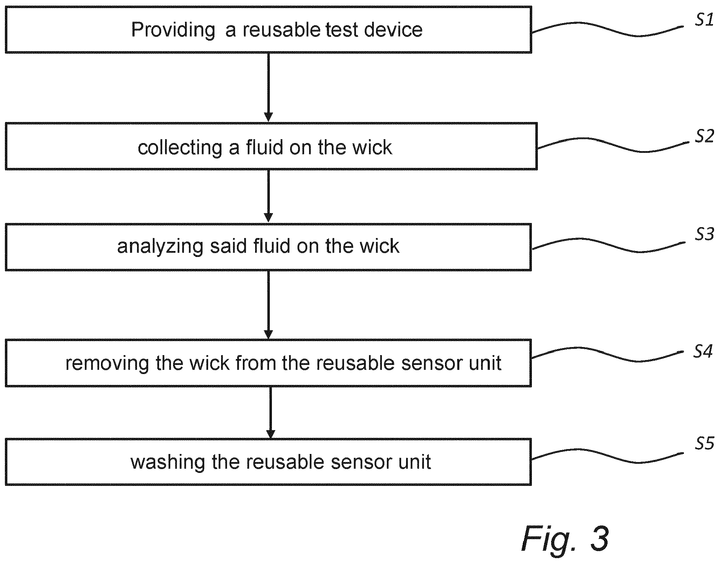

Fig. 3 illustrates an exemplary process flow chart of a method for testing the presence of an analyte 10 in a fluid. In a first step, step S1, a reusable test device 1, as illustrated in Fig 1a and b, is provided.

The reusable test device 1 may, in step S1, be provided in an assembled state, wherein the wick 2 is connected to the reusable sensor unit 3 which in turn is connected to the rechargeable electronic unit 4. In the next step, step S2, a fluid is collected by the wick 2. The fluid is then, in step S3, analyzed with the wick 2 attached to the reusable sensor unit 3 and the rechargeable electronic unit 4. After results have been read out, the wick 2 is preferably removed, step S4, from the reusable sensor unit 3, and the test device 1 is washed, step S5, with at least one washing and/or regeneration medium, as exemplified in Table 1. Preferably, the reusable sensor unit 3 is removed from the rechargeable electronic unit 4 prior to wash, thus allowing only the reusable sensor unit 3 to be washed. The reusable sensor unit 3 may then be reused up to 30 times, such as up to 20 times, or from 10 to 20 times.

In another embodiment, the wick 2 is not connected to the reusable sensor unit 3 when the sample is collected. In such embodiment, the wick is connected to the reusable sensor unit 3 prior to analyze in step S3.

The reusable sensor unit 3 or the test device 1 may be washed by a washing and/or regeneration medium comprising a liquid. The washing medium may for example comprise water which may or may not contain a surfactant and/or salts and be adjusted to different temperatures (see examples in Table 1).

The skilled person realizes that a number of modifications of the embodiments described herein are possible without departing from the scope of the disclosure, which is defined in the appended claims.

### Example 1. Synthesis of MIP nanogels for SV and PQ

A dispersed-phase approach described by Mahajan et al. (Mahajan et al. Angew. Chem. Int. Ed. 2019, 58, 727 -730) was adopted for MIP nanoparticle synthesis. The surface of magnetic nanoparticles was activated and then incubated in a solution of toluene with (3-aminopropyl)triethoxysilane followed by succinic anhydride.

The SV and PQ epitopes were then coupled through EDC/DMAP chemistry through their N-terminal modifications.

The monomer composition was adopted from the previously introduced nanogel imprinting protocol by Hoshino et al (Y. Hoshino, T. Kodama, Y. Okahata and K. J. Shea, J. Am. Chem. Soc. 2008, 130, 15242-15243). A prepolymerization mixture was prepared by dissolving NIPAM (39 mg, 344.64 µmol), BIS (2 mg, 12.97 µmol), TBAm (33 mg, 259.47 µmol dissolved in 1 mL ethanol), AA (2.2 µL, 31.92 µmol), APMA (5.80mg, 33.00µmol) and 2.6 mg of N-fluoresceinylacrylamide in water (50 mL) in a 250 mL round bottom flask. The solution was degassed under vacuum and sonicated for 10 minutes. Thereafter the peptide modified magnetic nanoparticles (magNP-SV or magNP-PQ) (50mg) dispersed in deionized water (10mL) was added into the flask. The flask was then sealed with a septum and purged with nitrogen for 20 minutes. The polymerization was then initiated by injecting a solution composed of APS (30 mg, 130 µmol) and TEMED (30 µL) in 1 ml deionized water under nitrogen atmosphere. The reaction solution was stirred on an orbital shaker at room temperature for 12 hours. The particles were then collected by magnet and washed with deionized water (10 x 20 mL) at room temperature until a clear solution was obtained. Finally the high-affinity nanoparticles were eluted by incubating the magnetic particles in deionized water (20 mL) at reduced temperature for 10 min under vigorous shaking. The elution was repeated five times resulting in a total volume of 100 mL of imprinted nanogel (PQ-NG or SV-NG) stock solution.

### Example 2. Displacement assay proving binding specificity for hCGbeta 66-80.

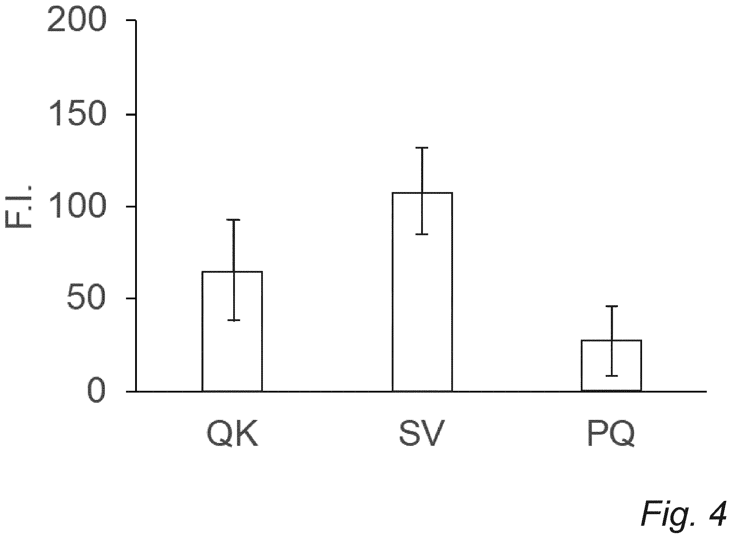

An aliquot (100 µL) of the SV-NG nanogel stock solution (vide supra) (≈ 0.1mg/ml) was mixed with an aliquot (50 µL) of magNP-SV in PBS buffer (0.1M, pH 7.2) followed by shaking for 2 hours. The displacement assay was then started by addition of peptide solutions (50µL, 100 nM in PBS buffer) and leaving the solutions to incubate for 2 hours. The fluorescence emission was recorded by top mode reading using 485nm/520nm excitation/emission filters (Figure 4).

### Example 3. SPR and QCM analysis of MIP-NG binding affinity for hCGbeta 66-80

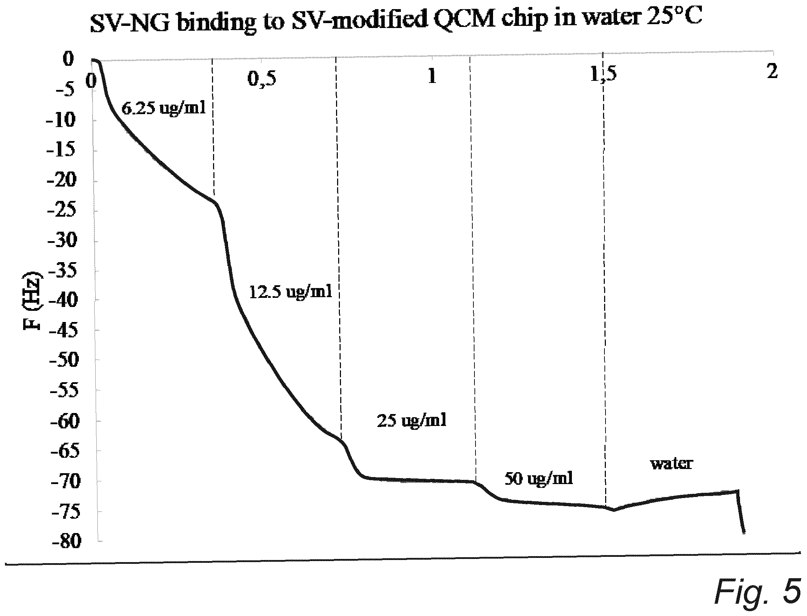

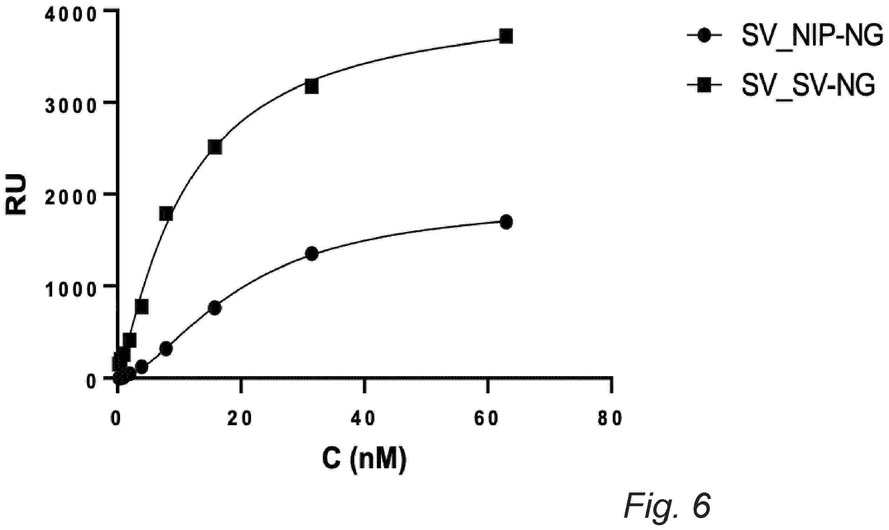

Analysis was performed on SPR gold chips (GE Healthcare, UK) modified with lipoic acid (Sigma Aldrich). Bare gold chips were first cleaned by hydrogen plasma and then placed in ethanol containing 0.3 mg/mL lipoic acid and 5 % (v/v) acetic acid overnight in a sealed vial. The chips were subsequently rinsed with ethanol and dried under a stream of N2 and then mounted in the SPR instrument (Biacore 3000, GE Healthcare, UK). The chips were then activated by injection of EDC/NHS in water followed by 5 injections of SV-NG, PQ-NG, or NIP-NG (0.1 mg mL-1) in water. The same activation procedure was used for the peptide immobilization. SV, PQ or hCG were then injected onto the MIP-modified chips and analysis performed using PBS at pH 7.4. For binding assays using peptide modified chips, the MIP-NGs were injected onto the chip with immobilized peptide. Example of resulting binding curves is seen in Fig. 6. Fig. 5 shows a corresponding experiment using the micro-gravimetric QCM technique.

### Example 4. SPR sensor chip regeneration.

Regeneration of the sensor surface after a sensing experiment as described in Example 3 was performed by repeated rinsing of the sensor with the respective buffer at the indicated temperatures followed by repeated rinsing in deionized water.

A net increase of 1500 RUs was measured when saturating an SV-modified SPR sensor chip with SV-MIP. Sequential injection of 200 µL of buffers 7 and 8 for 600 s each led to a decreased signal of 1472 Rus leading to complete regeneration of the sensor chip. The sensor could be repeatedly used with minimal loss of performance between runs.

Fig. 4 illustrates displacement of FITC-doped SV-imprinted MIP nanogel (SV-NG) bound to SV-modified magnetic nanoparticles (SV-magNP) upon addition of different peptides (SV (SEQ ID NO:1): SIRI,PGCPRGVNPVV; PQ (SEQ ID NO:2): PGPSDTPILPQ; QK (SEQ ID NO:3): QKSLSLSPGK).

Fig. 5 illustrates quartz crystal microbalance (QCM) frequency change during addition of SV-NG to an SV-modified sensor chip. Apparent dissociation constant, KD ≈ 1 nM.

Fig. 6 illustrates change in SPR response units (RUs) upon addition of an SV-NG and a nonimprinted NG (NIP-NG) to an SV modified sensor chip. The apparent dissociation constant was KD = 11 nM.

**Table 1. Examples of sensor regeneration conditions**

| **Sensor regeneration conditions** | | **Concentration** | **pH/Temperature** |
|---|---|---|---|
| | **Rinsing buffers** | | |
| 1 | Glycine buffer (GB) | 10 mM | 2.2 |
| 2 | Sodium acetate (SA) | 100 mM | 5.0 |
| 3 | Sodium bicarbonate (SB) | 100 mM | 9.2 |
| 4 | TWEEN 20 | 0.005 % | - |
| 5 | SDS | 0.1 % | - |
| 6 | Denaturing buffer (DB) | 100 mM | 10.0 |
| 7 | EtOH:H₂O ^{b} | 70/30 | - |
| 8 | EtOH:0.1M NaOH | 70/30 | - |

| | **Thermal regeneration** | | |
|---|---|---|---|
| 9 | EtOH:H₂O | 70/30 | 60°C |
| 10 | EtOH:H₂O | 70/30 | 5°C |

| | | | |
|---|---|---|---|
| a) Regeneration of the sensor surface is performed by repeated rinsing of the sensor with the respective buffer at the indicated temperatures followed by repeated rinsing in deionized water. b) A net increase of 1500 RUs was measured when saturating an SV-modified SPR sensor chip with SV-MIP. Sequential injection of 200 µL of buffers 7 and 8 for 600 s each led to a decreased signal of 1472 Rus leading to complete regeneration of the sensor chip. | | | |

**Table 2. Possible epitopes for generating MIPs featuring specificity for hCG**

| **Antigenic domain** | **Molecular localization (amino acid #)** |
|---|---|
| Beta-1+3 | beta-20-25 + 68-77 |
| Beta-ctp | beta-139-145 |
| Beta-2+alpha-1 | beta-45-48, alpha-13-22 |
| Alpha-2 | alpha-33-41 |

| | |
|---|---|
| Adopted from P. Berger, A.J. Lapthom / Molecular Immunology 76 (2016) 134-145 | |

## Claims

1. A reusable test device (1), comprising:
a) a wick (2) adapted to collect a fluid to be analyzed;
b) a reusable sensor unit (3), comprising:
a MIP layer (8) comprising at least one type of molecular imprinted polymers (MIPs) adapted to bind at least one analyte (10) present in said fluid;
an electrode layer (7) comprising at least one electrode (22),
wherein said reusable sensor unit (3) is regenerable upon cleansing, and
c) a rechargeable electronic unit (4) adapted to read out results from said reusable sensor unit (3);
wherein the wick (2) is detachably connected to the reusable sensor unit (3).

2. The reusable test device (1) according to claim 1, wherein said at least one analyte comprises a hormone, said hormone being selected from the group consisting of estrogen, progesterone, luteinizing hormone (LH), and human chorionic gonadotropin (hCG), such as hyperglycosylated human chorionic gonadotropin (hCG-H), in particular beta human chorionic gonadotropin (β-hCG).

3. The reusable test device (1) according to claim 1 or 2, wherein said MIP layer (8) comprises two or more types of molecular imprinted polymers, such as three or four types of molecular imprinted polymers.

4. The reusable test device (1) according to claim 3, wherein said MIP layer (8) comprises a first type of molecular imprinted polymers adapted to bind a first analyte, and a second type of molecular imprinted polymers, adapted to bind said first analyte and/or a second analyte.

5. The reusable test device (1) according to any one of claims 1 to 4, wherein said at least one type of molecular imprinted polymers further comprises a third type of molecular imprinted polymers adapted to bind any of said first, second and/or a third analyte.

6. The reusable test device (1) according to any one of claims 1 to 5, wherein said first analyte is hCG and said second analyte is glycosylated hCG or progesterone.

7. The reusable test device (1) according to any one of claims 1 to 6, wherein said fluid comprises a body fluid, such as saliva, blood, and urine, preferably urine

8. The reusable test device (1) according to any one of claims 1 to 7, wherein said wick (2) comprises a buffer solution, such as phosphate, Tris or citrate based buffer, such as wherein a concentration of said buffer solution is from 0.1 mM to 100 mM, such as 1 mM to 75 mM.

9. The reusable test device (1) according to any one of claims 1 to 8, wherein a physical property associated with the presence of an analyte in said MIP is a change in the resistance of said MIP layer (8) with or without said analyte bound.

10. The reusable test device (1) according to any one of claims 1 to 9, wherein said MIP layer (8) and said electrode layer (7) together form an integral layer.

11. The reusable test device (1) according to any one of claims 1 to 10, wherein said reusable sensor unit (3) is detachably connected to said rechargeable electronic unit (4).

12. Use of the reusable test device (1) according to any one of claims 1 to 11 for pregnancy testing.

13. A method for testing the presence of an analyte in a fluid, said method comprising the steps:
providing (S1) a reusable test device (1) according to claim 1;
collecting (S2) a fluid on said wick (2);
analyzing (S3) said fluid on said wick (2);
removing (S4) said wick from said reusable sensor unit (3), and
washing (S5) said reusable sensor unit (3) with at least one washing medium to remove any bound analytes (10) from said molecular imprinted polymers, wherein the steps S1 to S5 may be repeated.

14. The method according to claim 13, wherein said step of collecting is performed with said wick (2) connected to said reusable sensor unit (3), and with said reusable sensor unit (3) connected to said rechargeable electronic unit (4), in an assembled state.

15. Use of the method according to any one of claims 13 to 14 for pregnancy testing.

## Patentansprüche

1. Wiederverwendbares Testgerät (1), umfassend:
a) einen Docht (2), der dazu angepasst ist, ein zu analysierendes Fluid zu sammeln;
b) eine wiederverwendbare Sensoreinheit (3), umfassend:
eine MIP-Schicht (8), die mindestens einen Typ von molekularen geprägten Polymeren (MIPs) umfasst, die dazu angepasst sind, mindestens einen in dem Fluid vorhandenen Analyten (10) zu binden;
eine Elektrodenschicht (7), die mindestens eine Elektrode (22) umfasst, wobei die wiederverwendbare Sensoreinheit (3) beim Reinigen regenerierbar ist, und
c) eine wiederaufladbare Elektronikeinheit (4), die dazu angepasst ist, Ergebnisse aus der wiederverwendbaren Sensoreinheit (3) auszulesen;
wobei der Docht (2) lösbar mit der wiederverwendbaren Sensoreinheit (3) verbunden ist.

2. Wiederverwendbares Testgerät (1) nach Anspruch 1, wobei der mindestens eine Analyt ein Hormon umfasst, wobei das Hormon aus der Gruppe ausgewählt ist, die aus Östrogen, Progesteron, luteinisierendem Hormon (LH) und humanem Choriongonadotropin (hCG), wie hyperglykosyliertem humanem Choriongonadotropin (hCG-H), insbesondere humanem beta-Choriongonadotropin (β-hCG) besteht.

3. Wiederverwendbares Testgerät (1) nach Anspruch 1 oder 2, wobei die MIP-Schicht (8) zwei oder mehr Arten von molekularen geprägten Polymeren umfasst, wie drei oder vier Arten von molekularen geprägten Polymeren.

4. Wiederverwendbare Testvorrichtung (1) nach Anspruch 3, wobei die MIP-Schicht (8) einen ersten Typ von molekularen geprägten Polymeren umfasst, die dazu angepasst sind, einen ersten Analyten zu binden, und einen zweiten Typ von molekularen geprägten Polymeren, die dazu angepasst sind, den ersten Analyten und/oder einen zweiten Analyten zu binden.

5. Wiederverwendbares Testgerät (1) nach einem der Ansprüche 1 bis 4, wobei der mindestens eine Typ von molekularen imprägnierten Polymeren ferner einen dritten Typ von molekularen imprägnierten Polymeren umfasst, der dazu angepasst ist, einen des ersten, des zweiten und/oder eines dritten Analyten zu binden.

6. Wiederverwendbares Testgerät (1) nach einem der Ansprüche 1 bis 5, wobei der erste Analyt hCG ist und der zweite Analyt glykosyliertes hCG oder Progesteron ist.

7. Wiederverwendbares Testgerät (1) nach einem der Ansprüche 1 bis 6, wobei das Fluid eine Körperflüssigkeit, wie Speichel, Blut und Urin, bevorzugt Urin, umfasst.

8. Wiederverwendbares Testgerät (1) nach einem der Ansprüche 1 bis 7, wobei der Docht (2) eine Pufferlösung, wie etwa Puffer auf Phosphat-, Tris- oder Citrat-Basis, umfasst, wobei eine Konzentration der Pufferlösung von 0,1 mM bis 100 mM, wie etwa 1 mM bis 75 mM, beträgt.

9. Wiederverwendbares Testgerät (1) nach einem der Ansprüche 1 bis 8, wobei eine physikalische Eigenschaft, die mit der Anwesenheit eines Analyten in dem MIP assoziiert ist, eine Änderung des Widerstands der MIP-Schicht (8) mit oder ohne den Analyten gebunden ist.

10. Wiederverwendbares Testgerät (1) nach einem der Ansprüche 1 bis 9, wobei die MIP-Schicht (8) und die Elektrodenschicht (7) zusammen eine integrale Schicht bilden.

11. Wiederverwendbares Testgerät (1) nach einem der Ansprüche 1 bis 10, wobei die wiederverwendbare Sensoreinheit (3) lösbar mit der wiederaufladbaren Elektronikeinheit (4) verbunden ist.

12. Verwendung des wiederverwendbaren Testgeräts (1) nach einem der Ansprüche 1 bis 11 für Schwangerschaftstests.

13. Verfahren zum Testen der Anwesenheit eines Analyten in einem Fluid, wobei das Verfahren die Schritte umfasst:
Bereitstellen (S1) eines wiederverwendbaren Testgeräts (1) nach Anspruch 1;
Sammeln (S2) eines Fluids auf dem Docht (2);
Analysieren (S3) des Fluids auf dem Docht (2);
Entfernen (S4) des Dochts von der wiederverwendbaren Sensoreinheit (3), und
Waschen (S5) der wiederverwendbaren Sensoreinheit (3) mit mindestens einem Waschmedium, um gebundene Analyten (10) von den molekularen geprägten Polymeren zu entfernen, wobei die Schritte S1 bis S5 wiederholt werden können.

14. Verfahren nach Anspruch 13, wobei der Schritt des Sammelns mit dem Docht (2) mit der wiederverwendbaren Sensoreinheit (3) verbunden und mit der wiederverwendbaren Sensoreinheit (3) mit der wiederaufladbaren Elektronikeinheit (4) verbunden in einem zusammengebauten Zustand durchgeführt wird.

15. Verwendung des Verfahrens nach einem der Ansprüche 13 bis 14 für Schwangerschaftstests.

## Revendications

1. Appareil de test réutilisable (1), comprenant :
a) une mèche (2) adaptée pour recueillir un fluide à analyser ;
b) une unité de capteur réutilisable (3), comprenant :
une couche de MIP (8) comprenant au moins un type de polymères à empreintes moléculaires (MIP) adaptés pour lier au moins un analyte (10) présent dans ledit fluide ;
une couche d'électrode (7) comprenant au moins une électrode (22), dans lequel ladite unité de capteur réutilisable (3) est régénérable lors du nettoyage, et
c) une unité électronique rechargeable (4) adaptée pour lire les résultats de ladite unité de capteur réutilisable (3) ;
dans lequel la mèche (2) est reliée de manière amovible à l'unité de capteur réutilisable (3).

2. Appareil de test réutilisable (1) selon la revendication 1, dans lequel ledit au moins un analyte comprend une hormone, ladite hormone étant choisie parmi l'oestrogène, la progestérone, l'hormone lutéinisante (LH), et la gonadotrophine chorionique humaine (hCG), telle que la gonadotrophine chorionique humaine hyperglycosylée (hCG-H), en particulier la bêta-gonadotrophine chorionique humaine (β-hCG).

3. Appareil de test réutilisable (1) selon la revendication 1 ou 2, dans lequel ladite couche de MIP (8) comprend deux ou plusieurs types de polymères moléculaires imprimés, tels que trois ou quatre types de polymères moléculaires imprimés.

4. Appareil de test réutilisable (1) selon la revendication 3, dans lequel ladite couche de MIP (8) comprend un premier type de polymères moléculaires imprimés adaptés pour lier un premier analyte, et un deuxième type de polymères moléculaires imprimés adaptés pour lier ledit premier analyte et/ou un deuxième analyte.

5. Appareil de test réutilisable (1) selon l'une quelconque des revendications 1 à 4, dans lequel ledit au moins un type de polymères moléculaires imprimés comprend en outre un troisième type de polymères moléculaires imprimés adaptés pour se lier à l'un quelconque desdits premier, deuxième et/ou troisième analytes.

6. Appareil de test réutilisable (1) selon l'une quelconque des revendications 1 à 5, dans lequel ledit premier analyte est l'hCG et ledit deuxième analyte est l'hCG glycosylée ou la progestérone.

7. Appareil de test réutilisable (1) selon l'une quelconque des revendications 1 à 6, dans lequel ledit fluide comprend un fluide corporel, tel que la salive, le sang et l'urine, de préférence l'urine.

8. Appareil de test réutilisable (1) selon l'une quelconque des revendications 1 à 7, dans lequel ladite mèche (2) comprend une solution tampon, telle qu'un tampon à base de phosphate, Tris ou de citrate, telle que dans lequel la concentration de ladite solution tampon est de 0,1 mM à 100 mM, telle que 1 mM à 75 mM.

9. Appareil de test réutilisable (1) selon l'une quelconque des revendications 1 à 8, dans lequel une propriété physique associée à la présence d'un analyte dans ledit MIP est un changement de la résistance de ladite couche de MIP (8) avec ou sans liaison dudit analyte.

10. Appareil de test réutilisable (1) selon l'une quelconque des revendications 1 à 9, dans lequel ladite couche de MIP (8) et ladite couche d'électrode (7) forment ensemble une couche monobloc.

11. Appareil de test réutilisable (1) selon l'une quelconque des revendications 1 à 10, dans lequel ladite unité de capteur réutilisable (3) est reliée de manière amovible à ladite unité électronique rechargeable (4).

12. Utilisation de l'appareil de test réutilisable (1) selon l'une quelconque des revendications 1 à 11 pour les tests de grossesse.

13. Procédé de test de la présence d'un analyte dans un fluide, ledit procédé comprenant les étapes suivantes :
la fourniture (S1) d'un appareil de test réutilisable (1) selon la revendication 1 ;
la collecte (S2) d'un fluide sur ladite mèche (2) ;
l'analyse (S3) dudit fluide sur ladite mèche (2) ;
le retrait (S4) de ladite mèche de ladite unité de capteur réutilisable (3), et
le lavage (S5) de ladite unité de capteur réutilisable (3) avec au moins un milieu de lavage pour éliminer tout analyte lié (10) desdits polymères à empreintes moléculaires, dans lequel les étapes S1 à S5 peuvent être répétées.

14. Procédé selon la revendication 13, dans lequel ladite étape de collecte est réalisée avec ladite mèche (2) reliée à ladite unité de capteur réutilisable (3), et avec ladite unité de capteur réutilisable (3) reliée à ladite unité électronique rechargeable (4), dans un état assemblé.

15. Utilisation du procédé selon l'une quelconque des revendications 13 à 14 pour les tests de grossesse.
